# EUROPÄISCHE PATENTSCHRIFT

(11) **EP 1 847 227 B1**
(45) Veröffentlichungstag und Bekanntmachung des Hinweises auf die Patenterteilung: **12.11.2008**
(21) Anmeldenummer: 07006869.7
(22) Anmeldetag: 02.04.2007
(51) Int. Cl.: A61B 17/72, A61F 2/28, A61N 1/32

(54) **Elektrisches Marknagelsystem**
Electric medullar nail system
Système de clou électrique

(30) Priorität: 19.04.2006 DE 102006018191
(43) Veröffentlichungstag der Anmeldung: 24.10.2007
(73) Patentinhaber: Neue Magnetodyn GmbH, 80333 München (DE)
(72) Erfinder: Kraus, Werner, 80539 München (DE); Kraus, Stephanie, 83646 Bad Tölz (DE); Wiegmann, Markus, 81379 München (DE)
(74) Vertreter: Schumacher & Willsau

(56) Entgegenhaltungen:
- EP-A2- 0 781 532
- WO-A-02/38082
- DE-A1- 2 636 818
- DE-C1- 19 709 514
- US-B1- 6 224 601

## Beschreibung

Die Erfindung betrifft ein Marknagelsystem mit einem lang gestreckten, einen Hohlraum aufweisenden, zumindest abschnittsweise elektrisch leitfähigen Nagelkörper, einer Spulenanordnung, einer mit einem ersten Pol der Spulenanordnung verbundenen ersten Elektrode und einer mit einem zweiten Pol der Spulenanordnung verbundenen zweiten Elektrode.

Derartige Marknagelsysteme sind auf dem Gebiet der Osteosynthese bekannt. Die Osteosynthese dient der belastungsstabilen Fixation der Fragmente eines gebrochenen oder kranken Knochens in seiner unverletzten, natürlichen Form durch implantierte Schrauben, Stützplatten, Drähte, Knochenmarknägel und dergleichen, die im Allgemeinen aus nicht rostenden Stahl- oder Titanlegierungen gefertigt sind. Diese Osteosynthesemittel ermöglichen die rasche Mobilisierung des Patienten bei gleichzeitiger Ruhigstellung des lädierten Knochens, die eine unerlässliche Voraussetzung für seine Heilung ist.

Problematisch an der starren Fixierung durch die vergleichsweise unelastischen, gewebeverdrängenden Stützimplantate ist jedoch die Behinderung der biologischen Erholung vor allem durch den Verlust von Blutgefäßen und Nerven. Außerdem leidet mit zunehmender Implantationsdauer die biomechanische Qualität der Stützstruktur durch den partiellen Entzug ihrer Funktion. Mit dem Verlust an biologischer Kontrolle aber wächst die Gefahr der Infektion durch resistente Bakterien (MRSA = Multiresistenter Staphylococcus Aureus). Es wurde gezeigt, dass diese die Oberfläche von Metallimplantaten in Form eines adhärenten Biofilmes besiedeln können und mit einer Schleimhülle aus Polysacchariden dem Angriff von Antibiotika widerstehen.

Diesen Problemen kann im Rahmen der orthopädischen Chirurgie durch die magnetisch induzierte Elektro-Osteotherapie begegnet werden, beispielsweise unter Verwendung der eingangs genannten gattungsgemäßen Marknagelsysteme, wie beispielsweise in DE 26 36 818 C2 (Basis für den Oberbegriff des Anspruchs 1) beschrieben. Bei der Elektro-Osteotherapie werden in Osteosythesemitteln elektrische Wechselpotentiale niedriger Frequenz dadurch induziert, dass ein betroffener Körperteil einem magnetischen Wechselfeld ausgesetzt wird. Seit langem wurde in zahlreichen klinischen Anwendungen der verfahrensgemäßen Technik bei chronisch therapieresistenten, meist infizierten Knochendefekten, Zysten und Tumormetastasen sowie in kliniknahen experimentellen Studien gezeigt, dass mit der Verwendung der Osteosynthese-Implantate als Quellen extrem niederfrequenter sinusförmiger elektrischer Wechselpotentiale in der dem Stützmetall anliegenden Knochenregion ein optimaler Heilungseffekt erzielt wird.

Die Technik der Übertragung funktioniert nach dem Transformatorprinzip: Die verletzte oder kranke Körperregion wird von einem extrem niederfrequenten sinusförmig verlaufenden Magnetfeld mit einer Frequenz von ca. 1 bis 100 Hz - vorzugsweise von 4 bis 20 Hz - und einer magnetischen Flussdichte von 0,5 bis 5 mT (5 bis 50 Gauß) durchflutet, das durch einen Funktionsstromgenerator in einer oder mehreren - primären - äußeren Stromspulen erzeugt wird, in die das mit den Osteosynthesemitteln versehene Körperteil eingebracht wird. Diese extrem niederfrequenten elektromagnetischen Felder durchdringen weitgehend verlustfrei das Gewebe, einschließlich eventuelle Kleidung und einen Gipsverband, sowie die unmagnetischen (austenitischen) Stützmetalle der Osteosynthese. Im elektrischen Kontakt mit diesen wird eine - sekundäre - Spulenanordnung, der so genannte Übertrager, implantiert. Die in dem Übertrager induzierten Elektropotentiale werden so im Bereich der Knochenläsion sowie allgemein in dem an die Osteosynthesemittel angrenzenden Gewebe zur Wirkung gebracht.

Mit dieser Technik der induktiven Übertragung therapeutisch wirksamer Elektropotentiale auf die Bestandteile der Osteosynthese wird die Infektionsgefahr durch percutane Stromleitungen vermieden, und es können die Behandlungsparameter elektrische Spannung, Frequenz, Intensität, Signalform und die Behandlungszeit mit der indikationsspezifischen Programmierung eines Funktionsstrom-Generators des induzierenden Magnetfeldes bestimmt werden.

Der Erfindung liegt die Aufgabe zugrunde ein gattungsgemäßes Marknagelsystem insbesondere im Hinblick auf seine Handhabbarkeit und flexible Verwendbarkeit während der Operation, seine Stabilität, seine biologische Wirkung, seine therapeutische Wirksamkeit und seine Wirtschaftlichkeit zu verbessern.

Diese Aufgabe wird mit den Merkmalen des unabhängigen Anspruchs gelöst.

Vorteilhafte Ausführungsformen der Erfindung sind in den abhängigen Ansprüchen angegeben.

Die Erfindung baut auf dem gattungsgemäßen Marknagelsystem dadurch auf, dass die Spulenanordnung in einer proximal mit dem Nagelkörper lösbar verbundenen Endkappenanordnung mit einer zumindest teilweise elektrisch leitfähigen äußeren Kontaktoberfläche vorgesehen ist, dass die Kontaktoberfläche gegen den Nagelkörper elektrisch isoliert ist, dass zumindest ein Abschnitt der Kontaktoberfläche die erste Elektrode bildet und dass zumindest ein Abschnitt des Nagelkörpers die zweite Elektrode bildet. Während bei gattungsgemäßen Marknagelsystemen der Übertrager innerhalb des Nagelkörperhohlraums angeordnet ist, wählt die vorliegende Erfindung eine andere Anordnung, nämlich in einem Gehäuse einer Endkappe, die bei der Implantation abschließend mit dem Nagelkörper in Verbindung gebracht wird. Hierdurch kann der Nagelkörper unbeeinflusst von den elektrischen Komponenten implantiert werden. Insbesondere wird die Verwendung eines Führungsspießes nicht durch im Nagelkörperhohlraum angeordnete Bauteile behindert oder gar unmöglich gemacht. Der Führungsspieß wird in herkömmlicher Weise in den gebrochenen Knochen, beispielsweise die Tibia, eingeführt, und der Marknagel kann ohne weiteres nachgeführt werden. Anschließend wird der Führungsspieß entfernt, und es können distale und/oder proximale Verriegelungsschrauben appliziert werden, die den Nagel durch gegenüberliegende Öffnungen zur Erzielung von zusätzlicher Rotationsstabilität durchdringen. Zum Abschluss der Implantation wird die Endkappe, deren Gehäuse einen Pol der Spulenanordnung kontaktiert, mit dem Nagelkörper verbunden. Dabei wird insbesondere ein elektrischer Kontakt zwischen dem anderen Pol der Spulenanordnung und dem Nagelkörper hergestellt, so dass die Kontaktoberfläche der Endkappenanordnung und der Nagelkörper ein Elektrodenpaar bilden. Zusätzlich zu den Vorteilen im Hinblick auf die Applizierbarkeit eines Führungsspießes ist festzuhalten, dass der Nagelkörper durch etwaige Ausnehmungen zur Aufnahme elektrischer Komponenten, beispielsweise Ausfräsungen, nicht geschwächt wird. Folglich behält der Nagelkörper die Stabilität, die er auch im konventionellen "nicht elektrischen" Fall hätte, was zu einer erheblichen Reduzierung der Nagelbruchwahrscheinlichkeit führt. Diese Reduzierung wird noch dadurch erhöht, dass aufgrund der vorteilhaften Wirkung der elektrischen Potentiale der Heilungsprozess verkürzt wird. Auf der Grundlage der Erfindung erhält die Endkappe somit eine Doppelfunktion. Zum einen verhindert sie das Einwachsen von Bindegewebe und Knochen in den Nagelkörper, das die Explantation des Nagelkörpers erschweren würde. Zum anderen beherbergt die Endkappe die Komponenten, die dem Marknagelsystem die elektrischen Eigenschaften vermittelt. Neben den erwähnten Vorteilen im Hinblick auf die Verwendung eines weitgehend unveränderten Nagelkörpers ist weiterhin festzuhalten, dass der Chirurg während der Operation entscheiden kann, ob er den Nagelkörper mit einer normalen Endkappe oder einer mit den elektrischen Komponenten ausgestatteten Endkappe verschließt. Außerdem sind die Bereitstellung und die Lagerhaltung magnetisch induzierbarer Endkappen wesentlich weniger aufwendig und damit kostengünstiger als die Bereitstellung von magnetisch induzierbaren Nagelkörpern mit den erforderlichen unterschiedlichen Abmessungen. Weitere biologische Vorteile sind: Die Infektionsgefahr wird durch eine verstärkte Durchblutung und eine Immunreaktion des stimulierten Gewebes gemindert, die Antibiotikaresistenz des multiresistenten Staphylococcus Aureus (MRSA) wird überwunden, und die Adhärenz von Bakterienfilmen auf der Oberfläche des Nagelkörpers wird durch die magnetisch induzierte elektrische Aktivierung der Oberfläche vermieden.

Die Erfindung ist in vorteilhafter Weise dadurch weitergebildet, dass die Endkappenanordnung ein elektrisch leitfähiges Endkappengehäuse aufweist, dessen Oberfläche die Kontaktoberfläche bildet. Beispielsweise kann das Endkappengehäuse aus demselben Material bestehen wie der Nagelkörper. Die in dem Endkappengehäuse angeordneten elektrischen Komponenten sind vorzugsweise von einem gießfähigen elektrisch isolierenden Kunststoff umgeben, beispielsweise Epoxidharz.

Zusätzlich oder alternativ zu dem Epoxidharzverguss kann das proximale Ende des Endkappengehäuses durch einen e-lekrtisch leitfähigen oder isolierenden Deckel verschlossen werden. Es ist nicht erforderlich, die gesamte Oberfläche des elektrisch leitfähigen Endkappengehäuses als Elektrode zu realisieren. Bei einer vorzugsweise zumindest abschnittsweise zylindrischen Endkappenanordnung kann zum Beispiel eine den Zylindermantel umgebende Ringelektrode vorgesehen sein, die über eine Isolierschicht mit dem nicht als Elektrode wirkenden Teil des Endkappengehäuses verbunden ist. Zum Beispiel kann die Ringelektrode in das Endkappengehäuse eingelassen sein, so dass eine glatte äußere Oberfläche zur Verfügung gestellt wird.

Insbesondere dann, wenn das ganze Endkappengehäuse eine Elektrode bildet ist nützlicherweise vorgesehen, dass die Endkappenanordnung und der Nagelkörper unter Vermittlung einer Isolierschicht über Gewinde verbunden sind. Die Endkappe kann somit einschließlich ihres Gewindebereiches aus einem einheitlichen elektrisch leitenden Material gefertigt werden, was die Fertigung erleichtert und durch die Verwendung von Metallgewinden eine stabile Verbindung zwischen Nagelkörper und Endkappe gewährleistet. Die erforderliche Isolierung zwischen Endkappe und Nagelkörper wird durch eine Isolierschicht bereitgestellt, die entweder fest mit dem Nagelkörper oder fest mit der Endkappe verbunden ist. Ebenfalls ist es möglich, die Isolierschicht als separates Element vor dem Aufsetzen der Endkappe einzubringen. Unter Verzicht auf die Vorteile einer Endkappe aus einheitlichem Material ist es auch möglich, den das Gewinde tragenden Abschnitt der Endkappe aus einem isolierenden Material zu fertigen.

Gemäß einer alternativen Ausführungsform der Erfindung ist vorgesehen, dass die Endkappenanordnung ein elektrisch isolierendes Endkappengehäuse sowie einen das Endkappengehäuse verschließenden elektrisch leitfähigen Deckel aufweist, dessen Oberfläche die Kontaktoberfläche bildet. Als Material für das Endkappengehäuse kommt beispielsweise Polyethylen in Frage, etwa in der Art, wie es auch für Gelenkpfannen im Bereich der Endoprothetik zum Einsatz kommt.

Die Erfindung ist in besonders vorteilhafter Weise dadurch weitergebildet, dass der zweite Pol der Spule über einen elastischen elektrischen Kontakt mit einem in dem Hohlraum des Nagelkörpers angeordneten, elektrisch leitfähigen Einsatzelement verbunden ist, das elektrisch leitend mit dem Nagelkörper verbunden ist. Durch den elastischen elektrischen Kontakt über beispielsweise eine Spiralfeder, eine Blattfeder oder dergleichen wird eine gute elektrische Leitfähigkeit im Kontaktbereich sichergestellt. Vor dem Aufschrauben der Endkappe wird ein elektrisch leitfähiges Einsatzelement in den Nagelkörper eingeführt. Im Anschluss daran wird die Endkappe aufgeschraubt, und durch einen elastischen elektrischen Kontakt, der am distalen Ende der Endkappe vorzugsweise zentral angeordnet ist, wird die Kontaktierung des zweiten Pols der Spulenanordnung mit dem Nagelkörper hergestellt. Der Einsatzkörper ist so in dem Nagelkörper befestigt, dass zumindest eine axiale Verschiebung in distale Richtung behindert ist. Auf diese Weise bietet der Einsatzkörper die erforderliche Gegenkraft für die die elektrische Kontaktierung begünstigende Verformung des elastischen Kontaktes.

Beispielsweise kann vorgesehen sein, dass das Einsatzelement eine Kompressionsschraube ist, über die eine zwei gegenüberliegende Langlöcher in dem Nagelkörper durchdringende Schaftschraube mit einer axial gerichteten Kraft beaufschlagbar ist. Die Kompressionsschraube drückt gegen eine in den Langlöchern platzierte Schaftschraube, wodurch die Knochenfragmente im Bereich des Frakturspaltes aufeinander gepresst werden. In axial stabilen Frakturen entsteht so eine biomechanisch günstige, aktive, zirkumferente Kompression der Frakturfragmente. Insbesondere wird hierdurch die axiale Belastung auf den Knochen übertragen, so dass der Nagelkörper entlastet wird. Im Zusammenhang mit der vorliegenden Erfindung erhält die Kompressionsschraube eine Doppelfunktion. Neben der komprimierenden Funktionalität wird die Kompressionsschraube zum Bestandteil des elektrischen Systems, indem sie nämlich den Kontakt zwischen dem zweiten Pol der Spulenanordnung und dem als Elektrode wirkenden Nagelkörper herstellt.

Die Erfindung ist in besonders vorteilhafter Weise dadurch weitergebildet, dass die Spulenanordnung mit der Kontakoberfläche über einen elektrischen Gleichrichter in der Weise verbunden ist, dass die durch die Kontaktoberfläche gebildete erste Elektrode zumindest überwiegend positive Polarität hat. Auf diese Weise wird die magnetisch induzierte Osteogenese auf den Stabilisierungsbereich des Marknagelsystems, das heißt den Nagelkörper, konzentriert, da die Osteogenese von der Polarität der jeweiligen Elektroden abhängt, nämlich an der Kathode begünstigt und an der Anode behindert wird. Folglich wird die Knochenbildung in der Umgebung der Endkappe behindert, vermieden, und/oder es wird eine Osteolyse bewirkt, während im Bereich der Fraktur die Knochenbildung in erwünschter Weise verstärkt wird. Insbesondere wird hierdurch die Explantation des Marknagelsystems vereinfacht, denn die Endkappe kann zum Zwecke der Explantation einfach abgenommen werden, ohne dass dies durch Knochengewebe behindert würde. Durch die magnetisch induzierte Osteogenese in der Umgebung des Nagelkörpers wird die Wiederherstellung der mechanischen Belastbarkeit des Knochens beschleunigt. Dadurch kann die chirurgische Methode der Umwandlung einer statischen Verriegelung des heilenden Knochens in eine dynamische Verriegelung durch die Entfernung der proximalen Verriegelungsschrauben zu einem früheren Zeitpunkt geschehen. Dies gilt auch für den Zeitpunkt der Entnahme des gesamten Marknagelsystems.

Es kann vorgesehen sein, dass in Parallelschaltung zu dem Gleichtrichter ein ohmscher Widerstand vorgesehen ist. Ebenfalls kann vorgesehen sein, dass in Parallelschaltung zu dem Gleichtrichter ein kapazitiver Widerstand vorgesehen ist. Durch diese Maßnahmen wird eine unvollständige Gleichrichtung erreicht, wodurch Parameter zur Einstellung der geeigneten Verhältnisse im Hinblick auf die Osteogenese und die Osteolyse zur Verfügung stehen.

Nützlicherweise ist vorgesehen, dass die Spulenanordnung einen Spulenkern aufweist. Durch einen solchen Kern, beispielsweise einen weichmagnetischen Ferritkern, kann die elektrische Leistung bei gegebener äußerer Magnetfeldstärke erhöht werden. Unter Beibehaltung der elektrischen Leistung kann mit niedrigeren Magnetfeldstärken und/oder kleineren Komponenten in der Endkappe gearbeitet werden.

Weiterhin kann vorgesehen sein, dass in den Nagelkörper mindestens ein lang gestrecktes weichmagnetisches Element eingeschoben ist. Durch die Anordnung von weichmagnetischem Material im Nagelkörper wird das von außen applizierte Magnetfeld verstärkt. Diese Verstärkung wirkt sich auch im Bereich der Endkappe aus, so dass bei gegebener Übertragergröße höhere elektrische Leistungen zur Verfügung stehen. Bei gegebenem magnetischem Wechselfeld kann somit eine gewünschte elektrische Leistung unter Verwendung eines kleineren Übertragers zur Verfügung gestellt werden, so dass weniger Bauraum für den Übertrager benötigt wird. Das erfindungsgemäße Marknagelsystem kann daher mit kleinen Endkappen realisiert werden.

Gemäß einer weiteren bevorzugten Ausführungsform kann vorgesehen sein, dass in den Nagelkörper mindestens ein lang gestrecktes, ungesättigtes permanentmagnetisches Element eingeschoben ist. Die über die Oberflächenelektroden am Nagelkörper und an der Endkappe erzeugten elektrischen Felder haben eine nur sehr geringe Eindringtiefe in das umgebende Gewebe, die meist nur wenige Zelldurchmesser beträgt. Durch die Bereitstellung eines permanentmagnetischen Elementes wird ein Magnetfeld auch in weiter vom Implantat entfernten Geweberegionen erzeugt, wobei dieses mit zunehmender radialer Entfernung vom permanentmagnetischen Element abnimmt. Aufgrund der Anwesenheit dieses Gradientenmagnetfeldes können aufgrund der Bewegung des Gewebes elektrische Felder im Gewebe induziert werden, und zwar mit deutlich größerem Abstand vom Implantat als dies auf der Grundlage der Oberflächenelektroden möglich ist. Folglich kann auch in größerer Entfernung vom Implantat der Heilungsprozess gefördert werden. Das permanentmagnetische Element ist magnetisch ungesättigt, so dass dessen Magnetisierung dem von außen angewandten Wechselfeld teilweise folgen kann. Hierdurch wird sichergestellt, dass keine unerwünschte vollständige Konzentration des von außen angelegten Magnetfeldes auf den Bereich um das permanentmagnetische Element stattfindet. Vielmehr kann ein ausreichendes Magnetfeld im Bereich des Übertragers in der Endkappe zur Verfügung gestellt werden. Das ungesättigte permanentmagnetische Element kann somit auch vorteilhaft in Kombination mit einem weichmagnetischen Element eingeschoben werden.

Nützlicherweise ist vorgesehen, dass das mindestens eine lang gestreckte Element von einem Isoliermantel umgeben ist. Dieses kann beispielsweise durch einen Schrumpfschlauch gebildet sein, der das Element flüssigkeits- und gasdicht einhüllt.

Ebenfalls kann vorgesehen sein, dass mehrere lang gestreckte Elemente von demselben Isoliermantel umgeben sind. Werden beispielsweise mehrere weichmagnetische Elemente eingeschoben oder mehrere ungesättigte permanentmagnetische Elemente oder auch Kombinationen derselben, so können diese sogleich in einem einzigen Isoliermantel zusammengefasst sein. Das Einschieben ist während der Operation daher mit einem einzigen Handgriff bewerkstelligt.

Die Erfindung ist ferner dadurch besonders nützlich weitergebildet, dass die äußere Oberfläche des Nagelkörpers zumindest teilweise mit einer die Oberfläche des Nagelkörpers vergrößernden und die Anlagerung von Bakterien vermeidenden, elektrisch leitfähigen Beschichtung versehen ist. Bakterizide Beschichtungen sind bekannt. Wählt man eine elektrisch leitfähige bakterizide Beschichtung, die die Oberfläche des Nagelkörpers vergrößert, so kommt es zu einer Verstärkung des bakteriziden Effektes, nämlich aufgrund der vergrößerten Oberfläche zur Übertragung des elektrischen Feldes auf das umgebende Gewebe.

In diesem Zusammenhang ist bevorzugt, dass die Beschichtung Silber aufweist. Eine Silberbeschichtung kann beispielsweise direkt auf Implantate aus Stahl- oder Titanlegierungen mittels einer Sputtertechnik aufgebracht werden.

Nützlicherweise kann aber auch vorgesehen sein, dass zwischen der Oberfläche des Nagelkörpers und der Beschichtung eine poröse Zwischenschicht vorgesehen ist. Die elektrisch leitfähige Verbindung der Beschichtung mit der unter der Zwischenschicht liegenden Oberfläche des Nagelkörpers wird durch die umgebende Körperflüssigkeit und/oder durch direkten Kontakt der Silberpartikel mit der Oberfläche zur Verfügung gestellt. Die poröse Zwischenschicht besteht beispielsweise aus Keramik oder Kunststoff.

Es wird weiterhin ein Nagelkörper beschrieben, der geeignet ist, zusammen mit einem erfindungsgemäßen Marknagelsystem zum Einsatz zu kommen.

Ferner wird eine Endkappenanordnung gezeigt, die geeignet ist, zusammen mit dem erfindungsgemäßen Marknagelsystem zum Einsatz zu kommen.

Die Erfindung wird nun mit Bezug auf die begleitenden Zeichnungen anhand bevorzugter Ausführungsformen beispielhaft erläutert.

Es zeigen:
- Figur 1: eine Seitenansicht eines erfindungsgemäßen Marknagelsystems;
- Figur 2: einen in axiale Richtung geführten Schnitt durch den proximalen Endbereich einer ersten Ausführungsform eines erfindungsgemäßen Marknagelsystems;
- Figur 3: einen in axiale Richtung geführten Schnitt durch den proximalen Endbereich einer zweiten Ausführungsform eines erfindungsgemäßen Marknagelsystems;
- Figur 4: eine erste Ausführungsform einer im Zusammenhang mit der Erfindung einsetzbaren Gleichrichterschaltung;
- Figur 5: eine zweite Ausführungsform einer im Rahmen des erfindungsgemäßen Marknagelsystems einsetzbaren Gleichrichterschaltung;
- Figur 6: einen radialen Schnitt durch einen Nagelkörper eines erfindungsgemäßen Marknagelsystems mit darin angeordneten magnetischen Stäben und
- Figur 7: einen Schnitt durch die Oberfläche eines Nagelkörpers eines erfindungsgemäßen Marknagelsystems mit einer die Oberfläche vergrößernden Beschichtung.

Bei der nachfolgenden Beschreibung der bevorzugten Ausführungsformen der vorliegenden Erfindung bezeichnen gleiche Bezugszeichen gleiche oder vergleichbare Komponenten.

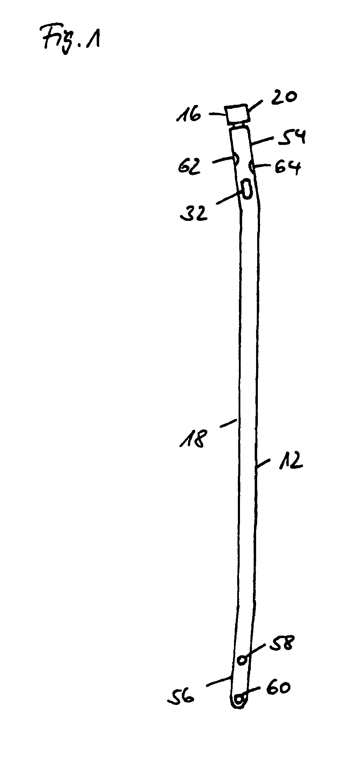

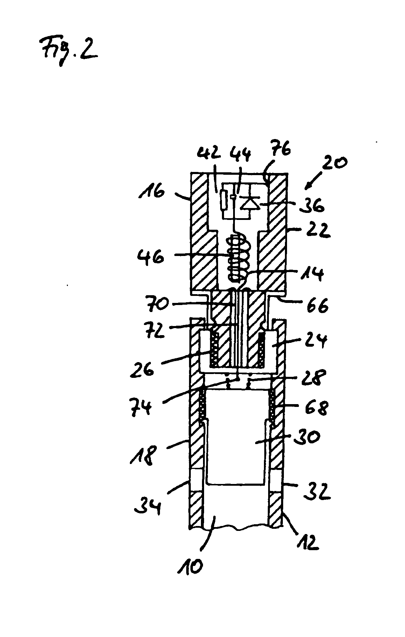

Figur 1 zeigt eine Seitenansicht eines erfindungsgemäßen Marknagelsystems; Figur 2 zeigt einen in axiale Richtung geführten Schnitt durch den proximalen Endbereich einer ersten Ausführungsform eines erfindungsgemäßen Marknagelsystems. Es ist ein Marknagelsystem für die Stabilisierung und Ruhestellung von Fragmenten eines gebrochenen Knochens dargestellt, beispielsweise des Schienbeinknochens (Tibia), des Oberschenkelknochens (Femur) oder des Oberarmknochens (Humerus). Das Marknagelsystem umfasst einen annähernd zylindrischen Nagelkörper 12 und eine Öffnung des Nagelkörper 12 an seinem proximalen Ende 54 verschließende im Wesentlichen axialsymmetrische Endkappenanordnung 20. Der Nagelkörper 12 hat an seinem distalen Ende 56 ebenfalls eine nicht dargestellte Öffnung. Die Öffnungen am proximalen Ende 54 und am distalen Ende 56 sind über einen Hohlraum 10 im Nagelkörper 12 miteinander verbunden. Es sind Verriegelungsöffnungen 58, 60, 62, 64 in der Wandung des Nagelkörpers vorgesehen, wobei den dargestellten Verriegelungsöffnungen 58, 60, 62, 64 jeweils eine weitere nicht dargestellte Verriegelungsöffnung diametral gegenüberliegt. Die eine Gruppe der Verriegelungsöffnungen 58, 60 ist am distalen Ende 56 angeordnet, während die andere Gruppe der Verriegelungsöffnungen 62, 64 am proximalen Ende 54 vorgesehen ist. Ebenfalls am proximalen Ende 54 des Nagelkörpers 12 ist ein sich diametral gegenüberliegendes Langlochpaar 32, 34 vorgesehen.

Das in Figur 1 dargestellte Marknagelsystem kommt im Rahmen der Osteosynthese wie folgt zum Einsatz. Zunächst wird ein (nicht dargestellter) Führungsspieß in den Hohlraum eines frakturierten Röhrenknochens über den Frakturspalt hinaus eingeführt. Im Anschluss daran wird der Nagelkörper 12 über den Führungsspieß in den Röhrenknochen geführt. Nachfolgend kann der Führungsspieß entnommen werden. Über die Verriegelungsöffnungen 58, 60, 62, 64 können eine oder mehrere den Knochenschaft durchdringende Verriegelungsschrauben eingesetzt werden, die dem durch den Nagelkörper 12 stabilisierten Knochen zusätzliche Rotationsstabilität geben. Eine weitere Schaftschraube kann durch die Langlöcher 32 geführt werden. Diese dient der axialen Kompression des Frakturspaltes, indem nämlich vom proximalen Ende eine Kompressionsschraube 30 in ein Innengewinde des Nagelkörpers eingeschraubt wird, die sich an ihrem distalen Ende an der in den Langlöchern 32, 34 angeordneten Schaftschraube 32 abstützt. Zum Abschluss der Implantation wird eine Endkappenanordnung 20 auf den Nagelkörper 12 aufgesetzt, vorzugsweise über einen Gewindebereich 26, der von einem Außengewinde an der Endkappenanordnung 20 in einem Innengewinde des Nagelkörpers gebildet wird.

Wie insbesondere in Figur 2 erkennbar ist, enthält die Endkappenanordnung 20 eine Spulenanordnung 14, und im aufgeschraubten Zustand der Endkappenanordnung 20 wirkt diese selbst als Elektrode, während der Nagelkörper 12 die Gegenelektrode bildet. Die Spulenanordnung 14 ist in einem freien Volumen des Endkappengehäuses 22 angeordnet. Die Spulenanordnung 14 umgibt einen Weicheisenkern, der zur Verstärkung des von außen zugeführten magnetischen Wechselfeldes vorgesehen ist. Ein Pol der Spulenanordnung 14 tritt über eine Parallelschaltung aus Diode 36, ohmschem Widerstand 42 und kapazitivem Widerstand 44 mit einer Kontaktstelle 76 des Endkappengehäuses 22 in Kontakt. Die durch die Diode 36 realisierte Gleichrichterschaltung kann sich vorteilhaft auf die Lokalisierung des Knochenwachstums auswirken. So wird die Oberfläche des Endkappengehäuses 22 zur Anode, an der das Knochenwachstum gehemmt wird oder sogar Osteolyse stattfindet, während der Nagelkörper 12 zur Kathode wird, so dass insbesondere im Frakturbereich das Knochenwachstum gefördert wird. Die zu der Diode 36 parallel geschalteten Bauteile, das heißt der ohmsche Widerstand 42 und der kapazitive Widerstand 44 sind optional. Durch sie wird der Spannungsverlauf im Vergleich zur nicht gleichgerichteten Spannung in Richtung positiver Polarität in der Weise verschoben, dass eine nicht vollständige Gleichrichtung vorliegt. Unter Verzicht auf die genannten Vorteile der Gleichrichtung ist die Diode entbehrlich, so dass der erste Pol der Spulenanordnung 14 das Endkappengehäuse 22 direkt kontaktieren kann. Der andere Pol der Spulenanordnung 14 befindet sich über eine Kontaktstelle 74 mit einer Spiralfeder 28 in elektrischem Kontakt. Zu diesem Zweck wird eine elektrische Leitung 72 durch einen distalen Bereich des Endkappengehäuses geführt, wobei eine Isolierung 70 einen elektrischen Kurzschluss der Spulenanordnung verhindert. Der distale Bereich der Endkappenanordnung 20, der gegenüber dem proximalen Bereich verjüngt ist, trägt ein Gewinde. Über einen Gewindebereich 26 ist die Endkappenanordnung 20 in den Nagelkörper 12 eingeschraubt, wobei eine den Kurzschluss der Spulenanordnung verhindernde Isolierschicht 24 vorgesehen ist. Diese Isolierschicht 24 setzt sich vorteilhaft in proximale Richtung fort, beispielsweise bis zur Isolierung 66 beim Übergang zwischen dem proximalen zu dem distalen Bereich der Endkappenanordnung 20. In den Nagelkörper 12 ist weiterhin eine Kompressionsschraube 30 über einen Gewindebereich 68 eingeschraubt. Wie erwähnt dient diese Kompressionsschraube 30 dazu, eine die Langlöcher 32, 34 durchdringende Schaftschraube in axiale Richtung mit Kraft zu beaufschlagen, so dass eine Kompression im Bereich des Frakturspaltes erfolgen kann. Im vorliegenden Zusammenhang dient die Kompressionsschraube 30 weiterhin zur elektrischen Kontaktierung der Spiralfeder 28, die sich an ihrem proximalen Ende an der Isolierung 24 und an ihrem distalen Ende an der Kompressionsschraube 30 abstützt. Über den Gewindebereich 68 und gegebenenfalls über die die Langlöcher 32, 34 durchdringende, nicht dargestellte Schaftschraube wird der elektrische Kontakt zwischen der Spulenanordnung 14, das heißt insbesondere der Kontaktstelle 74, und dem Nagelkörper 12 hergestellt. Die elektrischen Komponenten im Inneren des Endkappengehäuses 22 sind von einer biologisch verträglichen Epoxidgießharzmasse elektrisch isolierend und mechanisch stabilisierend umgeben.

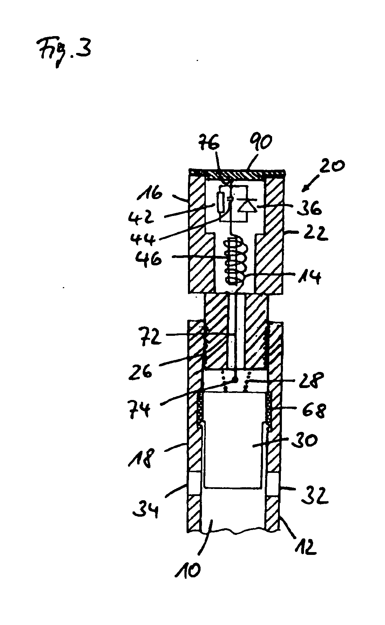

Figur 3 zeigt einen in axiale Richtung geführten Schnitt durch den proximalen Endbereich einer zweiten Ausführungsform eines erfindungsgemäßen Marknagelsystems. Im Unterschied zu der Ausführungsform gemäß Figur 2 kommt hier eine Endkappengehäuse 22 aus elektrisch isolierendem Material, beispielsweise biologisch verträglichem Polyethylen, zum Einsatz. Die Kontaktoberfläche wird durch einen elektrisch leitfähigen Deckel 90 gebildet, der das Endkappengehäuse 22 an seinem proximalen Ende verschließt. Der Deckel 90 kann mit dem Endkappengehäuse durch Kleben, Schrauben, Klipsen oder dergleichen verbunden sein. Im Falle einer gas- und flüssigkeitsdichten Verbindung des Deckels 90 mit dem Endkappengehäuse 22 ist ein Ausgießen des Gehäuseinneren entbehrlich, wenngleich dennoch möglich, beispielsweise zur mechanischen Stabilisierung der elektrischen Komponenten und Verbindungen. Die im Zusammenhang mit Figur 2 beschriebenen Isolierungen 24, 26, 70 des Endkappengehäuses 22 gegen den Nagelkörper sind bei elektrisch isolierendem Endkappengehäuse 22 gemäß Figur 3 entbehrlich.

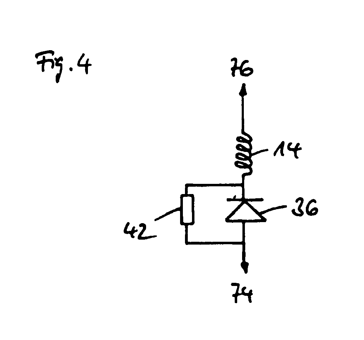

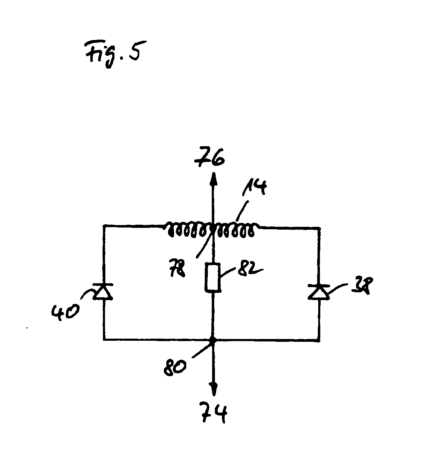

Die Figuren 4 und 5 zeigen zwei Ausführungsformen einer in Zusammenhang mit der Erfindung einsetzbaren Gleichrichterschaltung. Die Schaltung in Figur 4 entspricht im Wesentlichen der bereits anhand von Figur 2 beschriebenen Schaltung, wobei allerdings kein kapazitiver Widerstand vorliegt. Ebenso kann die Parallelschaltung eines ohmschen Widerstandes 62 je nach Anwendung entbehrlich sein. Während Figur 4 eine Einweggleichrichterschaltung zeigt, ist in Figur 5 eine Zweiweggleichrichtung dargestellt. Die Spulenanordnung 14 hat einen Mittelabgriff 78 von dem aus sie über einen ohmschen Widerstand 82 mit einem zur Kontaktstelle 74 am Nagelkörper 12 beziehungsweise der Spiralfeder 28 führenden Schaltungsknoten 80 verbunden ist. Der Mittelabgriff 78 ist weiterhin direkt mit der Kontaktstelle 76 an dem Endkappengehäuse verbunden. Am Schaltungsknoten 80 sind zwei Dioden 38, 40 angeschlossen, die die beiden Endpunkte der Spulenanordnung kontaktieren. Ebenso wie im Zusammenhang mit den Figuren 2 und 3 erläutert wurde, kann auch die in Figur 5 dargestellte Zweiweggleichrichterschaltung durch das Wechselstromverhalten der Schaltung beeinflussende Widerstände modifiziert werden.

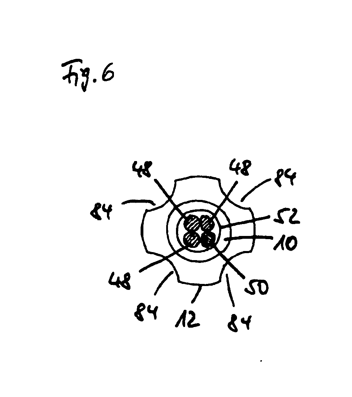

Figur 6 zeigt einen radialen Schnitt durch einen Nagelkörper eines erfindungsgemäßen Marknagelsystems mit darin angeordneten magnetischen Stäben. Der Nagelkörper 12 hat mehrere entlang seines Umfangs angeordnete sich in axiale Richtung erstreckende Ausnehmungen 84, die eine Rotationsstabilisierung des Nagelkörpers 12 im Knochen zur Verfügung stellen. Im Hohlraum 10 des Nagelkörpers 12 ist ein Isoliermantel 52 mit vier darin angeordneten Stäben 48, 50 vorgesehen. Im vorliegenden Beispiel handelt es sich um drei Stäbe 48 aus weichmagnetischem Material und um einen Stab 50 aus ungesättigtem permanentmagnetischem Material. Andere Varianten sind möglich, nämlich eine Variation der Anzahl der Stäbe, ein ausschließliches Vorsehen von weichmagnetischem Material oder ein ausschließliches Vorsehen von ungesättigtem permanentmagnetischem Material. Die weichmagnetischen Stäbe 48 bündeln das von außen zugeführte magnetische Wechselfeld, so dass es zu einer lokalen Verstärkung kommt, die bis in den Bereich der in der Endkappenanordnung 20 vorgesehenen Spulenanordnung 14 wirkt. Folglich haben die weichmagnetischen Stäbe 48 eine verstärkende Wirkung auf die über die Gewebeelektroden zur Verfügung gestellte elektrische Leistung. Der ungesättigte permanentmagnetische Stab 50 kann dem extern zugeführten magnetischen Wechselfeld teilweise folgen, so dass, anders als bei einem gesättigten permanentmagnetischen Stab, ein "Kurzschluss" des Magnetfeldes verhindert wird. Die besondere Wirkung des permanentmagnetischen Elementes kommt bei fehlendem externem Magnetfeld zum Tragen, nämlich durch die Bereitstellung eines in den den Nagelkörper 12 umgebenden Gewebebereich eindringenden radial nach außen abnehmenden magnetischen Gradientenfeldes. Auf der Grundlage dieses permanent vorhandenen Magnetfeldes und der Gewebebewegungen senkrecht zum permanenten Magnetfeld werden elektrische Felder im Gewebe induziert, die den Heilungsprozess fördern. Im Gegensatz zu dem nur wenige Zelldurchmesser in das Gewebe eindringenden elektrischen Feld, das durch die Oberflächenelektroden erzeugt wird, dringt das Permanentmagnetfeld tief in das Gewebe ein, so dass auch hier den Heilungsprozess fördernde elektrische Felder induziert werden. Ein äußeres magnetisches Wechselfeld kann den Permanentmagneten zusätzlich in eine sich vorteilhaft auf den Heilungsprozess auswirkende Vibration versetzen.

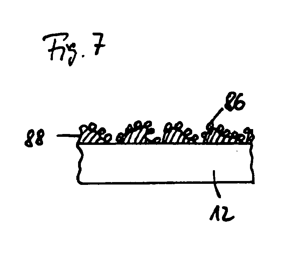

Figur 7 zeigt einen Schnitt durch die Oberfläche eines Nagelkörpers eines erfindungsgemäßen Marknagelsystems mit einer die Oberfläche vergrößernden Beschichtung. Die äußere Oberfläche des Nagelkörpers 12 ist mit einer die Oberfläche vergrößernden und die Anlagerung von Bakterien vermeidenden elektrisch leitfähigen Beschichtung, vorzugsweise aus in kolloidem Zustand vorliegenden Silberpartikeln 26, versehen. Die Beschichtung der Oberfläche wird durch eine poröse Zwischenschicht 68 vermittelt, die beispielsweise aus Kunststoff oder Keramik besteht. Ebenfalls ist möglich, dass die Silberpartikel zusätzlich oder alternativ in die Zwischenschicht eingelagert sind. Dies kann durch das Aufbringen einer Keramik-Silber-Emulsion realisiert werden. Der elektrische Kontakt zwischen der Oberfläche des Nagelkörpers 12 und der elektrisch leitfähigen Beschichtung 86 wird über Körperflüssigkeit oder durch direkten Kontakt der Oberfläche des Nagelkörpers 12 mit der Beschichtung 86 im Bereich der Poren der porösen Oberfläche 88 zur Verfügung gestellt. Durch die bakterizide Beschichtung 86 wird die Anlagerung von Bakterien auch ohne über die Nageloberfläche zur Verfügung gestellte elektrische Potentiale behindert. Dieser Effekt wird im Rahmen der vorliegenden Erfindung durch die induzierten elektrischen Felder verstärkt. Weiterhin wird auch die Wirkung des induzierten elektrischen Feldes auf das umgebende Gewebe begünstigt, da die elektrisch leitfähige Beschichtung 86 die Kontaktoberfläche zwischen Gewebe und Elektrode vergrößert. Im Ergebnis können hierdurch die positiven biologischen Effekte verbessert werden, oder es können unter Beibehaltung einer gegebenen Qualität einfachere und kleinere Geräte zum Einsatz kommen, was insbesondere die Spulenanordnung und die das externe magnetische Wechselfeld erzeugenden Gerätschaften betrifft.

Die in der vorstehenden Beschreibung, in den Zeichnungen sowie in den Ansprüchen offenbarten Merkmale der Erfindung können sowohl einzeln als auch in beliebiger Kombination für die Verwirklichung der Erfindung wesentlich sein.

### Bezugszeichenliste

- 10: Hohlraum
- 12: Nagelkörper
- 14: Spulenanordnung
- 16: erste Elektrode
- 18: zweite Elektrode
- 20: Endkappenanordnung
- 22: Endkappengehäuse
- 24: Isolierschicht
- 26: Gewindebereich
- 28: elastischer elektrischer Kontakt
- 30: Kompressionsschraube
- 32: Langloch
- 34: Langloch
- 36: Diode
- 38: Diode
- 40: Diode
- 42: ohmscher Widerstand
- 44: kapazitiver Widerstand
- 46: Spulenkern
- 48: weichmagnetischer Stab
- 50: ungesättigtes permanentmagnetisches Element
- 52: Isoliermantel
- 54: proximales Ende
- 56: distales Ende
- 58: Verriegelungsöffnung
- 60: Verriegelungsöffnung
- 62: Verriegelungsöffnung
- 64: Verriegelungsöffnung
- 66: Isolierung
- 68: Gewindebereich
- 70: Isolierung
- 72: elektrische Leitung
- 74: Kontaktstelle
- 76: Kontaktstelle
- 78: Mittelabgriff
- 80: Schaltungsknoten
- 82: ohmscher Widerstand
- 84: Ausnehmungen
- 86: elektrisch leitfähige Beschichtung
- 88: poröse Zwischenschicht
- 90: Deckel

## Patentansprüche

1. Marknagelsystem mit einem lang gestreckten, einen Hohlraum (10) aufweisenden, zumindest abschnittsweise elektrisch leitfähigen Nagelkörper (12), einer Spulenanordnung (14), einer mit einem ersten Pol der Spulenanordnung verbundenen ersten Elektrode (16) und einer mit einem zweiten Pol der Spulenanordnung verbundenen zweiten Elektrode (18), **dadurch gekennzeichnet,**
- **dass** die Spulenanordnung (18) in einer proximal mit dem Nagelkörper (12) lösbar verbundenen Endkappenanordnung (20) mit einer zumindest teilweise elektrisch leitfähigen äußeren Kontaktoberfläche vorgesehen ist,
- **dass** die Kontaktoberfläche gegen den Nagelkörper elektrisch isoliert ist,
- **dass** zumindest ein Abschnitt der Kontaktoberfläche die erste Elektrode (16) bildet und
- **dass** zumindest ein Abschnitt des Nagelkörpers die zweite Elektrode (18) bildet.

2. Marknagelsystem nach Anspruch 1, **dadurch gekennzeichnet, dass** die Endkappenanordnung (20) ein elektrisch leitfähiges Endkappengehäuse (22) aufweist, dessen Oberfläche die Kontaktoberfläche bildet.

3. Marknagelsystem nach Anspruch 1 oder 2, **dadurch gekennzeichnet, dass** die Endkappenanordnung (20) und der Nagelkörper (12) unter Vermittlung einer Isolierschicht (24) über Gewinde (26) verbunden sind.

4. Marknagelsystem nach Anspruch 1, **dadurch gekennzeichnet, dass** die Endkappenanordnung (20) ein elektrisch isolierendes Endkappengehäuse (22) sowie einen das Endkappengehäuse verschließenden elektrisch leitfähigen Deckel (90) aufweist, dessen Oberfläche die Kontaktoberfläche bildet.

5. Marknagelsystem nach einem der vorangehenden Ansprüche, **dadurch gekennzeichnet, dass** der zweite Pol der Spule über einen elastischen elektrischen Kontakt (28) mit einem in dem Hohlraum (10) des Nagelkörpers (12) angeordneten, elektrisch leitfähigen Einsatzelement (30) verbunden ist, das elektrisch leitend mit dem Nagelkörper (12) verbunden ist.

6. Marknagelsystem nach Anspruch 5, **dadurch gekennzeichnet, dass** das Einsatzelement eine Kompressionsschraube (30) ist, über die eine zwei gegenüberliegende Langlöcher (32, 34) in dem Nagelkörper durchdringende Schaftschraube mit einer axial gerichteten Kraft beaufschlagbar ist.

7. Marknagelsystem nach einem der vorangehenden Ansprüche, **dadurch gekennzeichnet, dass** die Spulenanordnung (14) mit der Kontaktoberfläche über einen elektrischen Gleichrichter (36, 38, 40) in der Weise verbunden ist, dass die durch die Kontaktoberfläche gebildete erste Elektrode (16) zumindest überwiegend positive Polarität hat.

8. Marknagelsystem nach Anspruch 7, **dadurch gekennzeichnet, dass** in Parallelschaltung zu dem Gleichtrichter (36, 38, 40) ein ohmscher Widerstand (42) vorgesehen ist.

9. Marknagelsystem nach Anspruch 7 oder 8, **dadurch gekennzeichnet, dass** in Parallelschaltung zu dem Gleichtrichter (36, 38, 40) ein kapazitiver Widerstand (44) vorgesehen ist.

10. Marknagelsystem nach einem der vorangehenden Ansprüche, **dadurch gekennzeichnet, dass** die Spulenanordnung (14) einen Spulenkern (46) aufweist.

11. Marknagelsystem nach einem der vorangehenden Ansprüche, **dadurch gekennzeichnet, dass** in den Nagelkörper (12) mindestens ein lang gestrecktes weichmagnetisches Element (48) eingeschoben ist.

12. Marknagelsystem nach einem der vorangehenden Ansprüche, **dadurch gekennzeichnet, dass** in den Nagelkörper (12) mindestens ein lang gestrecktes, ungesättigtes permanentmagnetisches Element (50) eingeschoben ist.

13. Marknagelsystem nach Anspruch 11 oder 12, **dadurch gekennzeichnet, dass** das mindestens eine lang gestreckte Element (48, 50) von einem Isoliermantel (52) umgeben ist.

14. Marknagelsystem nach Anspruch 11 oder 12, **dadurch gekennzeichnet, dass** mehrere lang gestreckte Elemente (48, 50) von demselben Isoliermantel (52) umgeben sind.

15. Marknagelsystem nach einem der vorangehenden Ansprüche, **dadurch gekennzeichnet, dass** die äußere Oberfläche des Nagelkörpers (12) zumindest teilweise mit einer die Oberfläche des Nagelkörpers vergrößernden und die Anlagerung von Bakterien vermeidenden, elektrisch leitfähigen Beschichtung versehen ist.

16. Marknagelsystem nach Anspruch 15, **dadurch gekennzeichnet, dass** die Beschichtung Silber aufweist.

17. Marknagelsystem nach Anspruch 15 oder 16, **dadurch gekennzeichnet, dass** zwischen der Oberfläche des Nagelkörpers (12) und der Beschichtung eine poröse Zwischenschicht vorgesehen ist.

## Claims

1. An intramedullary nail system including an elongated nail member (12) comprising a cavity (10) and which is electrically conductive at least in part, a coil assembly (14), a first electrode (16) connected to a first pole of the coil assembly, and a second electrode (18) connected to a second pole of the coil assembly, **characterized in that**
- the coil assembly (18) is provided in an end cap assembly (20) proximally releasably connected to the nail member (12) and which has an at least partially electrically conductive outer contact surface,
- the contact surface is electrically insulated from the nail member,
- at least one section of the contact surface forms the first electrode (16) and
- at least one section of the nail member forms the second electrode (18).

2. The intramedullary nail system as set forth in claim 1, **characterized in that** the end cap assembly (20) features an electrically conductive end cap housing (22) the surface of which forms the contact surface.

3. The intramedullary nail system as set forth in claim 1 or 2, **characterized in that** the end cap assembly (20) and the nail member (12) are connected via threads (26) by means of an insulating layer (24).

4. The intramedullary nail system as set forth in claim 1, **characterized in that** the end cap assembly (20) comprises an electrically insulating end cap housing (22) as well as an electrically conductive cover (90) closing off the end cap housing, the surface of the cover forming the contact surface.

5. The intramedullary nail system as set forth in any of the preceding claims, **characterized in that** the second pole of the coil is connected via an elastic electrical contact (28) to an electrically conductive insert element (30) arranged in the cavity (10) of the nail member (12) and which is connected to the nail member (12) so as to establish an electrically conductive contact.

6. The intramedullary nail system as set forth in claim 5, **characterized in that** the insert element is a compression screw (30) via which a stud penetrating two opposite slots (32, 34) in the nail member can be subjected to an axially directed force.

7. The intramedullary nail system as set forth in any of the preceding claims, **characterized in that** the coil assembly (14) is connected to the contact surface via an electric rectifier (36, 38, 40) in such a way that the first electrode (16) formed by the contact surface has an at least predominantly positive polarity.

8. The intramedullary nail system as set forth in claim 7, **characterized in that** an ohmic resistor (42) is provided connected in parallel to the rectifier (36, 38, 40).

9. The intramedullary nail system as set forth in claim 7 or 8, **characterized in that** a capacitive resistor (44) is provided connected in parallel to the rectifier (36, 38, 40).

10. The intramedullary nail system as set forth in any of the preceding claims, **characterized in that** the coil assembly (14) comprises a coil core (46).

11. The intramedullary nail system as set forth in any of the preceding claims, **characterized in that** at least one elongated soft magnetic element (48) is inserted into the nail member (12).

12. The intramedullary nail system as set forth in any of the preceding claims, **characterized in that** at least one elongated unsaturated permanent magnetic element (50) is inserted into the nail member (12).

13. The intramedullary nail system as set forth in claim 11 or 12, **characterized in that** the at least one elongated element (48, 50) is surrounded by an insulating sheath (52).

14. The intramedullary nail system as set forth in claim 11 or 12, **characterized in that** several elongated elements (48, 50) are surrounded by one and the same insulating sheath (52).

15. The intramedullary nail system as set forth in any of the preceding claims, **characterized in that** the outer surface of the nail member (12) is provided at least in part with an electrically conductive coating enlarging the surface of the nail member and avoiding bacterial colonization.

16. The intramedullary nail system as set forth in claim 15, **characterized in that** the coating comprises silver.

17. The intramedullary nail system as set forth in claim 15 or 16, **characterized in that** a porous interlayer is provided between the surface of the nail member (12) and the coating.

## Revendications

1. Système de clou intramédullaire comportant un corps de clou (12) allongé qui comporte une cavité et qui est conducteur électrique au moins en partie, un agencement de bobine (14), une première électrode (16) reliée à un premier pôle de l'agencement de bobine et une deuxième électrode (18) reliée à un deuxième pôle de l'agencement de bobine, **caractérisé en ce que**
- l'agencement de bobine (18) est prévu dans un agencement d'embouts (20) relié proximalement au corps de clou (12) de manière détachable et ayant une surface extérieure de contact au moins partiellement conducteur électrique,
- la surface de contact est isolée électriquement contre le corps de clou,
- au moins une partie de la surface de contact forme la première électrode (16) et
- au moins une partie du corps de clou forme la deuxième électrode (18).

2. Système de clou intramédullaire selon la revendication 1, **caractérisé en ce que** l'agencement d'embouts (20) comporte un boîtier d'embouts (22) conducteur électrique dont la surface forme la surface de contact.

3. Système de clou intramédullaire selon la revendication 1 ou 2, **caractérisé en ce que** l'agencement d'embouts (20) et le corps de clou (12) sont reliés au moyen de filets (26) par l'intermédiaire d'une couche isolante (24).

4. Système de clou intramédullaire selon la revendication 1, **caractérisé en ce que** l'agencement d'embouts (20) comporte un boîtier d'embouts (22) électriquement isolante ainsi qu'un couvercle (90) conducteur électrique fermant le boîtier d'embouts (22) et dont la surface forme la surface de contact.

5. Système de clou intramédullaire selon l'une des revendications précédentes, **caractérisé en ce que** le deuxième pôle de la bobine est relié par un contact électrique élastique (28) à un élément d'insert (30) conducteur électrique qui est agencé dans la cavité (10) du corps de clou (12) et qui est relié au corps de clou (12) de manière à assurer un contact conducteur électrique.

6. Système de clou intramédullaire selon la revendication 5, **caractérisé en ce que** l'élément d'insert est une vis de compression (30) par laquelle une vis sans tête qui traverse deux trous allongés (32, 34) opposés dans le corps de clou peut être assujettie à une force orientée axialement.

7. Système de clou intramédullaire selon l'une des revendications précédentes, **caractérisé en ce que** l'agencement de bobine (14) est relié à la surface de contact par un redresseur électrique (36, 38, 40) de manière à ce que la première électrode (16) formée par la surface de contact ait une polarité au moins prépondéramment positive.

8. Système de clou intramédullaire selon la revendication 7, **caractérisé en ce qu'**il comporte une résistance ohmique (42) connectée en parallèle au redresseur (36, 38, 40).

9. Système de clou intramédullaire selon la revendication 7 ou 8, **caractérisé en ce qu'**il comporte une résistance capacitive (44) connectée en parallèle au redresseur (36, 38, 40).

10. Système de clou intramédullaire selon l'une des revendications précédentes, **caractérisé en ce que** l'agencement de bobine (14) comporte un noyau de bobine (46).

11. Système de clou intramédullaire selon l'une des revendications précédentes, **caractérisé en ce qu'**au moins un élément magnétique doux allongé (48) est inséré dans le corps de clou (12).

12. Système de clou intramédullaire selon l'une des revendications précédentes, **caractérisé en ce qu'**au moins un élément magnétique permanent insaturé allongé (50) est inséré dans le corps de clou (12).

13. Système de clou intramédullaire selon la revendication 11 ou 12, **caractérisé en ce que** le au moins un élément allongé (48, 50) est entouré d'une gaine isolante (52).

14. Système de clou intramédullaire selon la revendication 11 ou 12, **caractérisé en ce que** plusieurs éléments (48, 50) allongés sont entourés de la même gaine isolante (52).

15. Système de clou intramédullaire selon l'une des revendications précédentes, **caractérisé en ce que** la surface extérieure du corps de clou (12) est pourvue au moins partiellement d'un revêtement conducteur électrique qui élargit la surface du corps de clou et évite la fixation de bactéries.

16. Système de clou intramédullaire selon la revendication 15, **caractérisé en ce que** le revêtement comporte de l'argent.

17. Système de clou intramédullaire selon la revendication 15 ou 16, **caractérisé en ce qu'**une couche intermédiaire poreuse est prévue entre la surface du corps de clou (12) et le revêtement.
